# FASCICULE DE BREVET EUROPEEN

(11) **EP 2 464 342 B1**
(45) Date de publication et mention de la délivrance du brevet: **26.02.2014**
(21) Numéro de dépôt: 10761043.8
(22) Date de dépôt: 11.08.2010
(51) Int. Cl.: A61K 9/16, A61K 9/20, A61K 9/48, A61K 47/02, A61K 31/485, A61K 31/165, A61K 31/19

(54) **FORMULATIONS PHARMACEUTIQUES CONTRE LE MÉSUSAGE DES MÉDICAMENTS**
PHARMAZEUTISCHE FORMULIERUNGEN GEGEN DROGENMISSBRAUCH
PHARMACEUTICAL FORMULATIONS AGAINST DRUG MISUSE

(30) Priorité: 12.08.2009 FR 0955642
(43) Date de publication de la demande: 20.06.2012
(73) Titulaire: Debregeas Et Associes Pharma, 75008 Paris (FR)
(72) Inventeur: SUPLIE, Pascal, F-27400 Montaure (FR); LEBON, Christophe, F-28260 Rouvres (FR)
(74) Mandataire: Gallochat, Alain
(86) Numéro de dépôt international: PCT/FR2010/051697
(87) Numéro de publication internationale: WO 2011/018583

(56) Documents cités:
- EP-A1- 0 253 684
- EP-A1- 2 044 932
- EP-B1- 2 046 300
- WO-A1-00/38649
- WO-A2-03/026621
- FR-A1- 2 829 932
- GB-A- 2 331 702

## Description

La présente invention a pour objet de nouvelles formulations pharmaceutiques destinées à limiter voire éviter le mésusage des médicaments.

Le mésusage des médicaments consiste en une utilisation détournée de ceux-ci. Ce phénomène est un problème majeur de santé publique et est en constante progression. Ce mésusage provient de l'ambivalence de certains principes actifs qui sont à la fois des substances médicamenteuses thérapeutiques et en même temps des drogues illicites.

Les objectifs recherchés lors de cette pratique de mésusage sont de plusieurs ordres, à savoir : recherche d'une performance intellectuelle ou physique ; recherche de plaisir (connotation festive, rave party) ; problème d'addiction consécutif à une première utilisation ; soulagement de symptômes physiques ou psychologiques ; dans certain cas asservissement chimique (abus sexuel sous l'emprise de drogue) et aussi quelquefois réel mésusage par mégarde ou mauvaise utilisation du médicament (incompréhension chez les personnes âgées).

Les conséquences de ces mésusages sont multiples et variées : simple effet secondaire, phénomène de nécrose tissulaire quand le mésusage concerne une administration locale, troubles cardiaques, dépression respiratoire, addiction voire même délit criminel (viol notamment).

Actuellement, les moyens disponibles sont peu nombreux et essentiellement administratifs : inscription des substances concernées dans des listes soumises à une législation particulière, délivrance par ordonnances sécurisées et délivrance en milieu hospitalier avec règles de délivrance restreintes. On observe également un effort des laboratoires sur le conditionnement de ces produits pour éviter notamment les ingestions accidentelles par les enfants.

Récemment, des études ont été effectuées au niveau galénique sur les formulations proprement dites.

Par exemple, dans le cas de comportement d'addiction ou de recherche d'asservissement chimique, les personnes concernées cherchant à détourner l'usage d'un médicament vont la plupart du temps soit ingérer directement le médicament, soit chercher à extraire le principe actif.

La première étape consiste alors à obtenir une poudre finement divisée de la composition médicamenteuse par broyage. Le principe actif est alors soit directement ingéré soit inhalé et le plus souvent extrait par solvant aqueux ou alcoolique.

Ainsi, la demande internationale WO03/013479 décrit l'association d'un analgésique opiacé avec son antagoniste qui bloque de façon significative l'effet euphorique de l'analgésique opioïde. Les deux composés sont libérés en même temps. Une telle formulation pharmaceutique comprenant un agoniste opiacé et son antagoniste présente toutefois des inconvénients. En effet, l'utilisation d'un antagoniste phamacologiquement actif peut être la cause de différents troubles.

La demande EP 1 293 209 décrit une forme à libération modifiée par l'utilisation d'une résine échangeuse d'ions ce qui permet de limiter l'extraction par mastication ou l'utilisation par inhalation ou injection. Toutefois, une telle formulation ne permet pas d'éviter une extraction par solvant ou par broyage.

La demande internationale WO2005/079760 décrit une formulation multiparticulaire utilisant un polymère plastique (Eudragit^{®} NE30D) qui, de par ses propriétés plastiques et l'utilisation concomitante d'un plastifiant, rend difficile le broyage de ladite formulation. Toutefois, pour cette formulation, aucun moyen permettant d'empêcher une extraction par solvant n'est prévu.

La demande internationale WO00/38649 décrit des comprimés bicouches destinés à éviter le détournement d'usage aux dépens d'un tiers, dotés de moyens visuels combinés constitués par des colorants, des générateurs d'effervescence et des composés gélifiables.

Ainsi, il existe donc à ce jour des formulations à libération prolongée utilisant différents artifices galéniques pour obtenir cette libération modifiée, mais aucun des systèmes décrits ne permet de se prémunir d'une extraction abusive du principe actif. Il est donc nécessaire de mettre au point une telle formulation.

La présente invention a donc pour but de fournir des formulations pharmaceutiques spécifiquement élaborées afin d'éviter l'utilisation détournée des médicaments.

La présente invention a pour but de fournir des formulations pharmaceutiques permettant de se prémunir d'une extraction abusive du principe actif.

La présente invention a pour but de fournir des compositions médicamenteuses, notamment orales, formulées spécifiquement pour assurer une plus grande sécurité face aux problèmes d'usage détourné des médicaments.

Un des buts de la présente invention consiste à fournir des formulations pharmaceutiques permettant de remédier et de compléter les dispositifs existants afin de diminuer et de rendre impossible les usages détournés de médicaments.

Un autre but de la présente invention consiste à fournir des formulations pharmaceutiques assurant l'effet thérapeutique recherché et seulement celui-ci.

Un autre but de la présente invention consiste à fournir des formulations pharmaceutiques facilement administrables et utilisables par leur forme et leur taille.

La présente invention concerne donc des granulés tels que définis dans les revendications.

Ainsi, la présente invention consiste à fournir des formulations pharmaceutiques comprenant l'utilisation de plusieurs artifices galéniques afin de rendre impossible chacune des techniques rencontrées lors des utilisations détournées. Ces formulations, à base des granulés susmentionnés, se présentent sous forme monolithique (comprimés) ou multiparticulaire. Lesdits granulés de l'invention comportent donc plusieurs couches de composition différente présentant chacune une fonctionnalité particulière.

L'expression "granulé" désigne une préparation constituée de grains solides secs, formant chacun un agrégat de particules de poudre d'une solidité suffisante pour permettre diverses manipulations.

Du point de vue physique, les granulés sont des agrégats de particules de poudres diverses cristallisées ou amorphes.

Les granulés de la présente invention sont notamment destinés à une administration par voie orale, et plus particulièrement à être avalés tels quels.

Les granulés de la présente invention présentent une structure caractéristique de type coeur-écorce, le coeur n'étant pas de même nature que les composés formant l'écorce.

Ainsi, ces granulés présentent une structure multicouche. En effet, le principe actif est déposé sur le coeur et donc forme une couche (ou écorce) déposée autour de ce coeur (ou support).

Le coeur des granulés peut également être considéré comme étant un support sur lequel vont se fixer les particules du principe actif.

Le coeur est constitué de particules solides et le principe actif supporté par ledit coeur est également sous forme solide.

La présente invention est donc basée sur la mise au point d'une nouvelle forme orale multiparticulaire.

Les granulés de l'invention présentent une couche de principe actif.

En fonction des paramètres pharmacologiques finaux souhaités, cette première couche peut être recouverte par d'autres couches polymériques utilisant différents polymères d'enrobage ainsi que les différents adjuvants couramment utilisés (plastifiants, solubilisants, lubrifiants, anti-adhérants, etc..).

Les granulés de l'invention comprennent un coeur solide choisi de préférence parmi les supports insolubles dans des solvants aqueux ou alcooliques. Le choix de ces supports insolubles pour former le coeur solide des granulés de l'invention permet d'éviter une solubilisation totale du granulé en cas de broyage.

Le coeur solide des granulés peut également être constitué d'un mélange de composés, notamment d'un mélange de supports insolubles. Ainsi, on peut notamment citer le mélange formé de saccharose et d'amidon ou de composés minéraux dérivés de la silice ou du calcium.

Le coeur solide peut également être constitué de supports solubles parmi lesquels on peut citer certains grades solides de PEG (notamment PEG 4000 ou PEG 6000).

L'expression "dérivés de la silice" désigne la silice ainsi que les silices précipitées obtenues à partir de silicates alcalins, notamment Aerosil^{®}, ou encore le talc, la bentonite ou le kaolin.

L'expression "dérivés de calcium" désigne des excipients cristallins dérivés de l'hydroxyde de calcium, des produits insolubles dans l'eau utilisés en médecine comme diluants, ou des agents de charges et également abrasifs.

L'expression "dérivés de potassium" désigne notamment le bicarbonate de potassium et le chlorure de potassium.

Parmi les supports insolubles formant le coeur des granulés de l'invention, on peut également citer les dérivés du magnésium (notamment carbonates ou oxydes).

Les granulés de l'invention comportent également un ou plusieurs agents colorants. Ainsi, on choisit les colorants en fonction de leurs solubilités dans les solvants. Par exemple, on choisit un colorant pour sa solubilité dans l'éthanol et un autre pour sa solubilité dans l'eau. En effet, ces deux solvants sont les solvants habituellement utilisés pour extraire ou solubiliser les principes actifs.

La coloration obtenue permet alors de visualiser les ajouts malveillants dans une boisson, par exemple pour soumission chimique.

Les granulés de l'invention comportent également un ou plusieurs pigments métalliques.

La présence de colorants et de pigments métalliques permet également de visualiser une solubilisation éventuelle après broyage de la forme pharmaceutique et par la suite une éventuelle ingestion. De même, en cas de mastication, on observe un phénomène identique.

Les colorants et les pigments métalliques peuvent être indifféremment placés dans les différentes couches des granulés de l'invention.

Avantageusement, on effectue un mélange intime du ou des colorants avec le principe actif et on utilise le pigment métallique dans la couche superficielle de la composition, c'est-à-dire celle qui est visible en surface.

Les granulés de l'invention comprennent également dans leur structure un ou plusieurs composés susceptibles de générer un dégagement gazeux lorsque la forme médicamenteuse se trouve hydratée. Ainsi, la présence d'un tel composé va provoquer, lors du versement des granulés dans un liquide, un mouvement vertical montant et descendant des granulés au fur et à mesure de la génération et du largage de dioxyde de carbone, avec en surface l'apparition d'une couche mousseuse.

Ce phénomène est encore plus flagrant quand la boisson utilisée est déjà gazeuse (soda/coca). En effet, on observe alors une véritable effervescence dans le verre.

L'utilisation de ces différentes solutions galéniques apportent à la forme pharmaceutique finale une sécurité maximale et garantit du détournement de cette forme.

Selon un mode préféré, les granulés susmentionnés comprennent également un liant.

Le rôle du liant est de lier entre elles les particules, c'est-à-dire de parfaire la cohésion du granulé. Ainsi, les liants permettent d'assurer une bonne cohésion du principe actif et du coeur dans les granulés.

Ainsi, les liants se trouvent, comme le principe actif, déposés autour du coeur des granulés.

Comme liants, on peut citer la plupart des excipients hydrophiles qui donnent des solutions visqueuses : gommes arabique et adragante, méthylcellulose et carboxyméthylcellulose, gélatine, amidons, maltodextrines, PEG 4000 et 6000 en solution alcoolique, polyvidone en solution aqueuse ou alcoolique, et aussi des solutions de saccharose, de glucose ou de sorbitol.

Les liants des granulés de l'invention sont de préférence choisis dans le groupe constitué de l'amidon, du saccharose, de la gomme arabique, de la polyvinylpyrrolidone (PVP ou polyvidone), de l'hydroxypropylméthylcellulose (HPMC), de la gomme laque, de l'hydroxypropylcellulose (HPC), de la cellulose, des polyols ou des alginates, des glycérides polyglycolysés (Gelucire^{®}) ou des macrogolglycérides, notamment macrogolglycérides de stéaroyle, également dérivés acryliques, ainsi que des mélanges de ceux-ci.

Parmi les polyols, on peut citer notamment le mannitol, le sorbitol, le maltitol ou le xylitol.

Selon un mode de réalisation particulier, les liants sont de préférence choisis dans le groupe constitué de la polyvinylpyrrolidone, de la gomme laque, des polyols ou des alginates, des glycérides polyglycolysés (Gelucire^{®}) ou des macrogolglycérides, notamment macrogolglycérides de stéaroyle, ainsi que des mélanges de ceux-ci.

On peut également utiliser un liant choisi dans les groupes cités ci-dessus pour des propriétés particulières ; par exemple il peut être utile d'utiliser comme liant des excipients pH-dépendants tels que EUDRAGIT^{®} L100 ou de la gomme laque. On peut également choisir d'utiliser préférentiellement des glycérides polyglycolysés (Gelucire^{®}) pour leur caractère hydrophobe.

Selon un mode de réalisation préféré, les granulés de l'invention comprennent en outre un ou plusieurs agents d'amertume.

De préférence, ledit agent d'amertume est choisi dans le groupe constitué du benzoate de dénatonium, des extraits de gentianes, de la quinine, de la caféine, de la brucine, de la quassine, du propyl thiouracile (PROP), du phénylthiocarbamide (PTC), des composés astringents tels que les tannins, des arômes de pamplemousse et des arômes de cacao amer.

La présence dudit agent d'amertume (ou promoteur d'amertume)(ex : Bitrex^{®}-benzoate de dénatonium) en mélange intime avec le principe actif rend difficile voire impossible une absorption par mastication accidentelle ou même après extraction et/ou solubilisation. En effet, ledit agent d'amertume se trouve alors solubilisé en même temps que le principe actif, la séparation différentielle étant très difficile.

L'utilisation d'un tel composé amer permet de prévenir les administrations volontaires ou dissimulées sous forme de "cocktails" dans des mélanges eau/alcool (glaçons/vodka etc...).

Parmi les composés susceptibles de générer un dégagement gazeux présents dans les granulés de l'invention, on peut notamment citer les composés choisis dans le groupe constitué des carbonates et bicarbonates.

Plus particulièrement, ces composés sont choisis dans le groupe constitué du bicarbonate de sodium, du carbonate de sodium, du carbonate de glycine de sodium, du bicarbonate de potassium, du carbonate de magnésium et du carbonate de calcium.

Parmi les agents colorants des granulés de l'invention, on peut citer notamment les agents colorants solubles dans des solvants aqueux et les agents colorants solubles dans des solvants alcooliques.

Parmi les agents colorants solubles dans l'éthanol, on peut notamment citer les colorants suivants : rouge neutre, brillant bleu FDC...

Parmi les agents colorants solubles dans l'eau, on utilise les colorants classiques alimentaires. Les colorants mis en oeuvre dans le cadre de la présente invention sont notamment ceux listés dans la directive 95/45/CE du 26 juillet 1995 concernant les colorants pouvant être employés dans les denrées alimentaires (modifiée par la directive 2006/33/CE du 20 mars 2006). Ainsi, on peut notamment citer les colorants E 100 à E 180.

On peut également citer le colorant E131 (bleu patenté) soluble à la fois dans l'eau et dans l'éthanol.

Selon un mode de réalisation particulièrement préféré, les pigments métalliques des granulés de l'invention sont des pigments à base de dioxyde de titane présents à la surface dudit granulé.

L'utilisation de ces pigments à base de titane (ex : CANDURIN^{®}) se révèle très utile pour les reflets métalliques qu'ils apportent, même en présence de boissons très foncées style Coca-Cola^{®}.

Les granulés enrobés sont constitués de grains enrobés d'une ou de plusieurs couches de mélanges d'excipients divers.

Ainsi, les granulés enrobés préférés selon la présente invention comprennent une couche supplémentaire constituée de l'agent d'enrobage.

Les granulés de l'invention peuvent également comprendre un enrobage constitué par un agent d'enrobage choisi dans le groupe constitué des dérivés de cire, des plastifiants (agents filmogènes), de la gomme laque, de la polyvinylpyrrolidone, du polyéthylène glycol, des dérivés cellulosiques tels que HPMC ou HPC, du saccharose, de l'alginate, des glycérides d'acides gras et des polymères méthacryliques.

L'expression "dérivés de cire" désigne des produits naturels ou synthétiques constitués d'esters d'acides gras et d'alcools, en général solides à température ambiante, utilisés à différents titres dans les préparations médicamenteuses.

Les granulés de l'invention peuvent également être enrobés par un film d'enrobage dans lequel sont ajoutés un ou plusieurs excipients tels que des lubrifiants, des colorants, des édulcorants, des plastifiants ou des agents anti-adhérants.

Les granulés de l'invention peuvent aussi comprendre un enrobage entérique, notamment constitué de polymères méthacryliques, notamment Eudragit^{®} L, de gomme laque ou de HPMCP (hydroxypropylméthyl cellulosephtalate - hypromellose phtalate).

De tels granulés sont donc gastro-résistants.

La présence de cet enrobage entérique peut influer sur la biodisponibilité du principe actif, notamment en évitant sa dégradation en milieu acide.

Les granulés de l'invention peuvent également comprendre un enrobage pour libération prolongée.

De tels granulés permettent une libération modifiée ou retardée des principes actifs (granulés à libération modifiée).

Un tel enrobage est obtenu avec des agents d'enrobage notamment constitués de copolymères de méthacrylates et d'acrylates Eudragit^{®} S100, de gomme laque, de dérivés de la cellulose, notamment d'éthylcellulose, et de dérivés acryliques.

La présence de cet enrobage pour libération modifiée influence notamment la demi-vie apparente du principe actif.

Les granulés selon la présente invention peuvent également comprendre un lubrifiant et/ou un arôme et/ou un édulcorant.

Parmi les lubrifiants mis en oeuvre dans le cadre de la présente invention, on peut notamment citer le talc, le stéarate de magnésium, les dérivés de silice (notamment Aerosil^{®}) ou les cires.

Parmi les arômes mis en oeuvre dans le cadre de la présente invention, on peut citer les arômes classiquement utilisés dans les additifs alimentaires.

Les édulcorants mis en oeuvre dans le cadre de la présente invention sont notamment ceux listés dans la directive 94/35/CE du 30 juin 1994 concernant les édulcorants destinés à être employés dans les denrées alimentaires (modifiée par la directive 2006/25/CE du 5 juillet 2006). Ainsi, on peut notamment citer l'aspartame E 951, le sorbitol E 420, le mannitol E 421, l'acésulfame-K E 950, la saccharine E 954, stevia ou la thaumatine.

Les granulés de l'invention peuvent comprendre tout principe actif utilisé en pharmacie thérapeutique, ainsi que des associations de ceux-ci. Parmi les principes actifs préférés, on peut citer les antalgiques et les analgésiques.

Les analgésiques permettent d'éliminer la douleur du patient. Parmi les classes d'analgésiques, on peut notamment citer les analgésiques centraux morphiniques (dérivés de morphine), les analgésiques centraux non morphiniques, les analgésiques périphériques et autres tels que les benzodiazépines.

De préférence, les principes actifs des granulés selon l'invention sont choisis dans le groupe constitué du sulfate de morphine, de l'oxycodone, de l'acide gamma-hydroxybutyrique ou l'un de ses sels, de la buprénorphine, du modafinil, du dextropropoxyphène, de la méthadone, du tramadol, de la nalbuphine, du tétrahydrocannabinol et des benzodiazépines.

Selon un mode de réalisation préféré, les granulés selon la présente invention ne comprennent pas d'antagoniste du principe actif. Les granulés de la présente invention présentent ainsi l'avantage de ne pas contenir d'agent modifiant l'activité thérapeutique du principe actif.

Lesdits granulés assurent donc l'effet thérapeutique recherché et seulement celui-ci, c'est-à-dire en n'utilisant pas d'autres composés actifs comme un antagoniste du principe actif.

Selon un autre mode de réalisation préféré, les granulés selon la présente invention ne comprennent pas de résine échangeuse d'ions.

De préférence, les granulés de l'invention comprennent de 0,5% à 60% en poids de principe actif par rapport au poids total du granulé.

De préférence, les granulés de l'invention comprennent de 0,2% à 4% en poids d'agent colorant par rapport au poids total du granulé.

De préférence, les granulés de l'invention comprennent de 0,1% à 5% en poids de pigments métalliques par rapport au poids total du granulé.

De préférence, les granulés de l'invention comprennent de 5% à 20% en poids de composés susceptibles de générer un dégagement gazeux par rapport au poids total du granulé.

De préférence, dans les granulés de l'invention, le coeur solide représente de 10% à 85% en poids par rapport au poids total du granulé.

La présente invention concerne également une composition pharmaceutique, comprenant des granulés tels que définis ci-dessus.

Aussi divulgué est un procédé de préparation d'un granulé tel que défini ci-dessus, caractérisé en ce qu'il comprend une étape d'application par poudrage du principe actif sur le support insoluble.

Selon un mode de réalisation préféré du procédé le principe actif est mélangé avec les agents colorants, les pigments métalliques et les composés susceptibles de générer un dégagement gazeux avant l'étape d'application par poudrage sur le support insoluble.

Le procédé peut également comprendre, après l'étape de poudrage, une étape d'enrobage du granulé, notamment en déposant par pelliculage l'agent enrobant sous forme de film sur le granulé, suivie, le cas échéant d'une étape de mélange avec un lubrifiant et/ou un arôme et/ou un édulcorant et/ou un colorant ou pigment métallique.

La structure des granulés de l'invention est liée à la mise en oeuvre de ce procédé particulier qui permet l'obtention de granulés de structure coeur-écorce.

En effectuant des essais comparatifs de préparation de granulés par un procédé de granulation directe avec différents excipients habituellement utilisés en granulation, il a été constaté que les résultats obtenus concernant le granulé lui-même sont satisfaisants concernant l'aspect, la friabilité et la dissolution. Cependant, les granulés obtenus par un tel procédé présentent une surface spécifique très élevée nécessitant des quantités importantes de polymères d'enrobage selon les techniques conventionnellement utilisées.

Ainsi, les granulés de la présente invention sont caractérisés en ce qu'ils présentent une surface spécifique abaissée. Par ailleurs, d'aspect, ils sont relativement lisses et présentent une forme plutôt régulière.

L'étape de poudrage susmentionnée du procédé pour la préparation des granulés de l'invention peut également comprendre une étape de pulvérisation d'une solution alcoolique ou hydroalcoolique ou aqueuse d'un liant.

Cette étape de pulvérisation et l'étape de poudrage sont de préférence effectuées de façon simultanée ou alternée.

De préférence, l'étape de poudrage susmentionnée est effectuée de façon concomitante avec une étape de pulvérisation d'un liant sous forme de solution.

La combinaison de ces étapes permet d'assurer une bonne cohésion du principe actif sur le coeur des granulés.

Une mise en oeuvre avantageuse du procédé consiste ainsi à appliquer le principe actif sous forme de poudre sur le support particulaire susmentionné (ou coeur des granulés) en alternant des séquences de pulvérisation du liant sous forme de solution.

Le procédé peut également comprendre, après l'étape de poudrage, une ou plusieurs étapes d'enrobage du granulé, notamment en déposant par pelliculage le ou les agents enrobants sous forme de films sur le granulé.

La faible surface spécifique des granulés de l'invention permet ainsi, en cas d'enrobage, de réduire la quantité utilisée d'agent d'enrobage et donc de moins diluer le principe actif dans lesdits granulés enrobés.

Un mode de réalisation préféré du procédé consiste en un procédé comprenant, après l'étape d'enrobage, une étape de mélange avec un lubrifiant et/ou un arôme et/ou un édulcorant, ceux-ci pouvant être eux-mêmes préparés sous forme de granulés afin d'être finalement mélangés aux granulés actifs.

Toutefois les lubrifiants, arômes et édulcorants peuvent être également ajoutés avant l'étape de poudrage susmentionnée.

### EXEMPLES

Les exemples ci-après concernent des exemples particuliers de formulations pharmaceutiques selon l'invention à base des granulés susmentionnés.

### Exemple 1: Granulés à base d'oxycodone

| *Granulés oxycodone 2 mg SR* | mg | % |
|---|---|---|
| **Matières Premières Sèches** | | |
| Oxycodone | 2,000 | 0,67 |
| Mannitol granulés | 131,44 | 43,81 |
| Cellulose microcristaline | 100,00 | 33,33 |
| Bitrex^{®} | 0,100 | 0,03 |
| Carbonate de glycine Na (CGS) | 15,000 | 5,00 |
| Povidone K30 | 9,000 | 3 |
| Mannitol 60 | 30,000 | 10,00 |
| Pigments | 0,500 | 0,17 |
| Colorant | 0,500 | 0,17 |
| Ethylcellulose | 9,000 | 3,00 |
| Huile de ricin hydrogénée | 2,160 | 0,72 |
| Talc | 0,300 | 0,10 |

| **Solvants** | | |
|---|---|---|
| Alcool 96° | Qs | |
| Eau purifiée | Qs | |
| | | |
| *Masse Théorique* | Qs | |
| *Masse Sèche Théorique* | 300,000 | 100,00 |
| *Teneur Théorique (mg*/*g)* | 6,67 | |

Les granulés susmentionnés sont obtenus en suivant le mode opératoire suivant :
Dans un premier temps, on a préparé une suspension active et ce en préparant une suspension aqueuse contenant le principe actif (oxycodone), le liant (PVP), le pigment et l'agent d'amertume (Bitrex^{®}).

Ladite suspension a été ensuite pulvérisée sur le support (granulés mannitol dans un lit d'air fluidisé) et les granules ont ensuite été séchés dans le lit d'air.

On a préparé un premier mélange (mélange 1) à partir de granulés de mannitol, de la cellulose microcristalline et du carbonate de glycine (composé générant un dégagement gazeux).

On a également préparé un deuxième mélange (mélange 2) à partir des granules mentionnés ci-dessus (obtenus à partir du principe actif). Ces granules ont été séchés puis placés dans le mélangeur avant introduction des colorants et des lubrifiants.

Enfin, les mélanges 1 et 2 ont ensuite été mélangés et calibrés.

### Exemple 2: Gélules à base de buprénorphine

| *Gélules buprénorphine 8 mg SR* | mg | % |
|---|---|---|
| **Matières Premières Sèches** | | |
| Buprénorphine | 8,000 | 2,00 |
| Neutres sucre amidon | 289,61 | 72,40 |
| Bitrex^{®} | 0,200 | 0,05 |
| Bicarbonate de Na | 26,250 | 6,56 |
| Ethylcellulose | 9,000 | 2,25 |
| Mannitol 60 | 30,000 | 7,50 |
| Pigments | 0,500 | 0,13 |
| Colorant | 0,500 | 0,13 |
| Aquacoat EC30D^{®} | 28,500 | 7,13 |
| Plastifiants | 6,840 | 1,71 |
| Talc | 0,600 | 0,15 |

| **Solvants** | | |
|---|---|---|
| Alcool 96° | Qs | |
| Eau purifiée | Qs | |
| | | |
| *Masse Théorique* | Qs | |
| *Masse Sèche Théorique* | 400,000 | 100,00 |
| *Teneur Théorique (mg*/*g)* | 20,00 | |

Les gélules susmentionnées sont obtenues en suivant le mode opératoire suivant :
Dans une turbine conventionnelle, on a placé les neutres supports (sucre/amidon) et on a préparé une suspension aqueuse contenant le principe actif (buprénorphine), le liant et l'agent d'amertume. Le bicarbonate de sodium (composé générant un dégagement gazeux) a ensuite été déposé par poudrage sur les supports de sucre et amidon en alternant les phases de poudrage et de pulvérisation de suspension.

Les granules obtenus ont ensuite été séchés en turbine.

On a ensuite effectué un enrobage avec une suspension aqueuse d'éthylcellulose comprenant le plastifiant, un colorant et du talc.

Les granules ont subi à nouveau une étape de séchage pour maturation du film.

Enfin, les granules ont ensuite été répartis en gélules.

### Exemple 3: Gélules à base de modafinil

| *Gélules modafinil 200 mg IR* | mg | % |
|---|---|---|
| **Matières Premières Sèches** | | |
| modafinil | 200,000 | 40,00 |
| Neutres cellulose | 187,86 | 37,57 |
| Bitrex^{®} | 0,200 | 0,04 |
| Carbonate de Na | 40,000 | 8,00 |
| Povidone | 30,000 | 6,50 |
| Pigments | 0,500 | 0,10 |
| Aérosil R972^{®} | 5,000 | 1 |
| Colorant | 0,500 | 0,10 |
| Polymère méthacrylique | 28,500 | 5,70 |
| Plastifiant | 6,840 | 1,37 |
| Talc | 0,600 | 0,12 |

| **Solvants** | | |
|---|---|---|
| Alcool 96° | Qs | |
| Eau purifiée | Qs | |
| | | |
| *Masse Théorique* | Qs | |
| *Masse Sèche Théorique* | 500,000 | 100,00 |
| *Teneur Théorique (mg*/*g)* | 400,00 | |

Les gélules susmentionnées sont obtenues en suivant le mode opératoire suivant :
Les supports de cellulose ont été placés dans une turbine conventionnelle.

Après micronisation, le principe actif a été mélangé au carbonate de sodium puis ce mélange a été appliqué par poudrage sur les supports en rotation dans la turbine.

On a ensuite préparé une solution avec le Bitrex^{®} et les pigments en les incorporant dans la solution liante alcoolique de PVP. Cette solution a ensuite été pulvérisée sur les supports en alternance avec les phases de poudrage.

Les granules obtenus ont ensuite été séchés de façon à éliminer les solvants utilisés pour l'étape de poudrage du principe actif.

La suspension d'enrobage a été préparée en préparant une suspension de polymères méthacryliques à laquelle on a ajouté le plastifiant, les lubrifiants et le colorant. Cette suspension a ensuite été placée sous agitation puis pulvérisée sur les granules obtenus précédemment.

Les granules enrobés ainsi obtenus ont ensuite été à nouveau séchés puis répartis en gélules.

### Exemple 4: Granules de GHB (acide gamma-hydroxybutyrique)

| *Ampoule GHB* | *g (quantité par ampoule)* | % |
|---|---|---|
| **Matières Premières Sèches** | | |
| GHB | 1,75 | 57,48 |
| Sphères de sucre | 0,61 | 11,48 |
| Bicarbonate de sodium | 0,26 | 8,52 |
| Shellac / gomme laque (E 904) | 0,20 | 6,56 |
| Neusilin UFL2^{®} | 0,16 | 5,25 |
| Sepifilm^{®} LP014 | 0,02 | 0,66 |
| Pharmacoat^{®} 603 | 0,03 | 0.98 |
| Talc | 0,02 | 0,66 |
| Agent d'amertume Bitrex^{®} | 0,2 | 6.56 |
| Colorant | 0,06 | 1,97 |

| **Solvants** | | |
|---|---|---|
| Alcool 96° | Qs | |
| Eau purifiée | Qs | |
| | | |
| *Masse Théorique* | Qs | |
| *Masse Sèche Théorique* | 3,05 | 100,00 |

Les microgranules de l'exemple 5 sont préparés en chargeant le principe actif (GHB) sur des sphères de sucre.

On a mélangé le principe actif GHB avec le bicarbonate de sodium et le composé Neusilin^{®} (aluminométasilicate de magnésium). Le mélange ainsi formé a été broyé.

En parallèle, une solution a été préparée avec la résine de shellac et l'éthanol puis celle-ci a été ajoutée au mélange susmentionné. On a alors également ajouté les sphères de sucre.

L'ensemble a ensuite été séché puis recouvert par un revêtement obtenu à partir d'un mélange formé des agents d'enrobage Sepifilm^{®} et Pharmacoat^{®} solubilisés dans de l'eau et de l'éthanol.

L'ensemble est ensuite de nouveau séché puis recouvert par un deuxième revêtement obtenu à partir d'un mélange formé de résine de shellac et de talc solubilisés dans de l'eau et de l'éthanol.

Enfin les granules ainsi obtenus ont été séchés avant ajout de talc comme lubrifiant.

## Revendications

1. Granulé ayant une structure du type coeur-écorce, ledit coeur étant choisi parmi les supports insolubles, et plus particulièrement choisi dans le groupe constitué des polyols, des gommes, des dérivés de la silice, des dérivés de calcium ou de potassium, des composés minéraux tels que les phosphates dicalciques, des phosphates tricalciques et des carbonates de calcium, du saccharose, des dérivés de cellulose, notamment de la cellulose microcristalline, de l'éthylcellulose et de l'hydroxypropylméthuylcellulose, de l'amidon, et des mélanges de ceux-ci, où ladite écorce comprenant un principe actif est d'une substance différente de celle dudit coeur et entoure ce dernier, ledit principe actif représentant entre 0,5% et 60% en poids par rapport au poids total dudit granulé, l'écorce de ce dernier comprenant en outre les composés suivants :
- un ou plusieurs agents colorants solubles dans des solvants aqueux ou alcooliques,
- un ou plusieurs pigments métalliques,
- un ou plusieurs composés susceptibles de générer un dégagement gazeux,
- et un ou plusieurs agents d'amertume.

2. Granulé selon la revendication 1, **caractérisé en ce que** le composé susceptible de générer un dégagement gazeux est choisi dans le groupe constitué des carbonates et bicarbonates, et est notamment choisi dans le groupe constitué du bicarbonate de sodium, du carbonate de sodium, du carbonate de glycine de sodium, du bicarbonate de potassium, du carbonate de magnésium et du carbonate de calcium.

3. Granulé selon l'une des revendications 1 ou 2, dans lequel les pigments métalliques sont des pigments à base de dioxyde de titane présents à la surface dudit granulé.

4. Granulé selon l'une quelconque des revendications 1 à 3, **caractérisé en ce que** le principe actif est choisi dans le groupe constitué des antalgiques et des analgésiques.

5. Granulé selon l'une quelconque des revendications 1 à 3, **caractérisé en ce que** le principe actif est choisi dans le groupe constitué du sulfate de morphine, de l'oxycodone, de l'acide gamma-hydroxybutyrique ou l'un de ses sels, de la buprénorphine, du modafinil, du dextropropoxyphène, de la méthadone, du tramadol, de la nalbuphine, du tétrahydrocannabinol et des benzodiazépines.

6. Granulé selon l'une quelconque des revendications 1 à 5, **caractérisé en ce que** l'agent d'amertume est choisi dans le groupe constitué du benzoate de dénatonium, des extraits de gentianes, de la quinine, de la caféine, de la brucine, de la quassine, du propyl thiouracile (PROP), du phénylthiocarbamide (PTC), des composés astringents tels que les tannins, des arômes de pamplemousse, et des arômes de cacao amer.

7. Granulé selon l'une quelconque des revendications 1 à 6, **caractérisé en ce qu'**il comprend de 0,2% à 4% en poids d'agent colorant, de 0,1% à 5% en poids de pigments métalliques et de 5% à 20% en poids de composés susceptibles de générer un dégagement gazeux par rapport au poids total du granulé.

8. Granulé selon l'une quelconque des revendications 1 à 7, **caractérisé en ce que** le coeur solide représente de 10% à 85% en poids par rapport au poids total du granulé.

9. Composition pharmaceutique comprenant des granulés selon l'une quelconque des revendications 1 à 8.

## Patentansprüche

1. Granulat mit einer Struktur vom Kern-Rinden-Typ, wobei der Kern aus den unlöslichen Trägern ausgewählt ist und insbesondere aus der Gruppe ausgewählt ist, die von den Polyolen, den Gummis, den Kieselerde-Derivaten, den Calcium- oder Kaliumderivaten, den mineralischen Verbindungen wie den Dicalciumphosphaten, den Tricalciumphosphaten und den Calciumcarbonaten, der Saccharose, den Zellulosederivaten, insbesondere der mikrokristallinen Zellulose, der Ethylzellulose und der Hydroxypropylmethylzellulose, der Stärke und den Gemischen derselben gebildet wird, wobei die Rinde, die ein Wirkprinzip umfasst, aus einer Substanz ist, die sich von der des Kerns unterscheidet und diesen umgibt, wobei das Wirkprinzip zwischen 0,5 und 60 Gew.-% im Verhältnis zum Gesamtgewicht des Granulats darstellt, wobei die Rinde desselben ferner die folgenden Verbindungen umfasst:
- ein oder mehrere Farbstoffe, die in wässrigen oder alkoholischen Lösungsmitteln löslich sind,
- ein oder mehrere metallische Pigmente,
- ein oder mehrere Verbindungen, die imstande sind, eine gasförmige Freisetzung zu erzeugen,
- und einen oder mehrere Bitterstoffe.

2. Granulat nach Anspruch 1, **dadurch gekennzeichnet, dass** die Verbindung, die imstande ist, eine gasförmige Freisetzung zu erzeugen, aus der Gruppe ausgewählt ist, die von den Carbonaten und Bicarbonaten gebildet wird und vor allem aus der Gruppe ausgewählt ist, die vom Natriumbicarbonat, vom Natriumcarbonat, vom Natriumglycincarbonat, vom Kaliumbicarbonat, vom Magnesiumcarbonat und vom Calciumcarbonat gebildet wird.

3. Granulat nach einem der Ansprüche 1 oder 2, wobei die metallischen Pigmente Pigmente auf der Basis von Titandioxid sind, die auf der Oberfläche des Granulats vorhanden sind.

4. Granulat nach einem der Ansprüche 1 bis 3, **dadurch gekennzeichnet, dass** das Wirkprinzip aus der Gruppe ausgewählt ist, die von den Antalgetika und Analgetika gebildet wird.

5. Granulat nach einem der Ansprüche 1 bis 3, **dadurch gekennzeichnet, dass** das Wirkprinzip aus der Gruppe ausgewählt ist, die von dem Morphinsulfat, dem Oxycodon, der Gamma-Hydroxybuttersäure oder einem ihrer Salze, dem Buprenorphin, dem Modafinil, dem Dextropropoxyphen, dem Methadon, dem Tramadol, dem Nalbuphin, dem Tetrahydrocannabinol und den Benzodiazepinen gebildet wird.

6. Granulat nach einem der Ansprüche 1 bis 5, **dadurch gekennzeichnet, dass** der Bitterstoff aus der Gruppe ausgewählt ist, die von dem Denatoniumbenzoat, den Enzianextakten, dem Chinin, dem Koffein, dem Brucin, dem Quassin, dem Propylthiouracil (PROP), dem Phenylthiocarbamid (PTC), den adstringierenden Bestandteilen wie den Tanninen, den Pampelmusenaromen und den Bitterkakaoaromen gebildet wird.

7. Granulat nach einem der Ansprüche 1 bis 6, **dadurch gekennzeichnet, dass** es im Verhältnis zum Gesamtgewicht des Granulats 0,2 bis 4 Gew.-% Farbstoff, 0,1 bis 5 Gew.-% metallische Pigmente und 5 bis 20 Gew.-% Verbindungen umfasst, die imstande sind, eine gasförmige Freisetzung zu erzeugen.

8. Granulat nach einem der Ansprüche 1 bis 7, **dadurch gekennzeichnet, dass** der feste Kern 10 bis 85 Gew.-% im Verhältnis zum Gesamtgewicht des Granulats darstellt.

9. Pharmazeutische Zusammensetzung, die Granulate nach einem der Ansprüche 1 bis 8 umfasst.

## Claims

1. A granule having a structure of the core-shell type, said core being selected from insoluble supports, and more particularly selected from the group consisting of polyols, gums, silica derivatives, calcium or potassium derivatives, mineral compounds such as dicalcium phosphates, tricalcium phosphates and calcium carbonates, saccharose, cellulose derivatives, notably microcrystalline cellulose, ethylcellulose and hydroxypropylmethylcellulose, starch, and mixtures thereof, wherein said shell comprising an active ingredient is of a substance different from that of said core and surrounds the latter, said active ingredient representing between 0.5% and 60% by weight based on the total weight of said granule, the shell of the latter futher comprising the following compounds:
- one or more coloring agents soluble in aqueous or alcoholic solvents,
- one or more metal pigments,
- one or more gas-releasing compounds,
- and one or more bitterness agents.

2. The granule according to claim 1, **characterized in that** the gas-releasing compound is selected from the group consisting of carbonates and bicarbonates, and is notably selected from the group consisting of sodium bicarbonate, sodium carbonate, sodium glycine carbonate, potassium bicarbonate, magnesium carbonate and calcium carbonate.

3. The granule according to one of claims 1 or 2, wherein the metal pigments are titanium dioxide-based pigments present at the surface of said granule.

4. The granule according to any of claims 1 to 3, **characterized in that** the active ingredient is selected from the group consisting of antalgics and analgesics.

5. The granule according to any of claims 1 to 3, **characterized in that** the active ingredient is selected from the group consisting of morphine sulfate, oxycodone, gamma-hydroxybutyric acid or one of its salts, buprenorphine, modafinil, dextropropoxyphene, methadone, tramadol, nalbuphine, tetrahydrocannabinol and benzodiazepines.

6. The granule according to any of claims 1 to 5, **characterized in that** the bitterness agent is selected from the group consisting of denatonium benzoate, gentian extracts, quinine, caffeine, brucine, quassin, propyl thiouracil (PROP), phenylthiocarbamide (PTC), astringent compounds such as tannins, grapefruit flavors, and bitter cocoa flavors.

7. The granule according to any of claims 1 to 6, **characterized in that** it comprises from 0.2% to 4% by weight of coloring agent, from 0.1% to 5% by weight of metal pigments and from 5% to 20% by weight of gas-releasing compounds based on the total weight of the granule.

8. The granule according to any of claims 1 to 7, **characterized in that** the solid core represents from 10% to 85% by weight based on the total weight of the granule.

9. A pharmaceutical composition comprising granules according to any of claims 1 to 8.
